# EUROPEAN PATENT SPECIFICATION

(11) **EP 2 053 407 B1**
(45) Date of publication and mention of the grant of the patent: **02.03.2011**
(21) Application number: 08386023.9
(22) Date of filing: 13.10.2008
(51) Int. Cl.: G01N 33/68

(54) **The protein serum amyloid P-component (SAP, SAMP) as prognostic and diagnostic marker for the prenatal diagnosis of Trisomy 21 (Down syndrome)**
Die Proteinserum-Amyloid P-Komponente (SAP, SAMP) als Prognose- und Diagnose-Marker für die pränatale Diagnose von Trisomie 21 (Down Syndrom)
Composant de la protéine amyloïde P (SAP, SAMP) du sérum en tant que marqueur de pronostic et de diagnostic pour le diagnostic prénatal de la trisomie 21 (syndrome de Down)

(30) Priority: 26.10.2007 GR 20070100651
(43) Date of publication of application: 29.04.2009
(73) Proprietor: Foundation of Biomedical Research of the Academy of Athens, 115 27 Athens (GR); Tsangaris, George, 152 35 Vrilissia Attikis (GR)
(72) Inventor: Tsangaris, George, 152 35 Vrilissia Attikis (GR); Vougas, Konstantinos, 155 61 Holargos Attikis (GR); Kolialexi, Aggeliki, 115 21 Athens (GR); Mavrou-Kalpini, Ariadni, 148 78 Ekali Attikis (GR); Antsaklis, Aristidis, 115 28 Athens (GR)
(74) Representative: Tefou-Fotea, Eleni

(56) References cited:
- WO-A-2005/021793
- US-A1- 2003 027 216
- US-A1- 2007 172 900
- HEEGAARD N H ET AL: "Ligand-binding sites in human serum amyloid P component." EUROPEAN JOURNAL OF BIOCHEMISTRY / FEBS 1 AUG 1996, vol. 239, no. 3, 1 August 1996 (1996-08-01), pages 850-856, XP002510940 ISSN: 0014-2956
- KOLIALEXI AGGELIKI ET AL: "Application of proteomics for the identification of differentially expressed protein markers for Down syndrome in maternal plasma." PRENATAL DIAGNOSIS AUG 2008, vol. 28, no. 8, August 2008 (2008-08), pages 691-698, XP002510941 ISSN: 0197-3851

## Description

### B. FIELD OF THE INVENTION

The present invention refers to the protein Serum amyloid P-component (SAP, SAMP) which could become a target and be used as a marker for the prenatal diagnosis and evaluation of trisomy 21 (Down syndrome) by non-invasive methods. More specifically, in the peripheral blood of women carrying foetuses with trisomy 21 (Down syndrome), Serum amyloid P-component (SAP, SAMP) is found to exhibit quantitative differences as compared to pregnants with chromosomally normal fetuses.

### C. BACKGROUND OF THE INVENTION

Today the methodology used for the prenatal diagnosis of fetal chromosomal abnormalities includes invasive and non-invasive techniques. Invasive procedures (amniocentesis, trophoblast biopsy and fetal blood) carry a significant risk for the mother and the fetus and for this reason they are recommended only in pregnant women in whom the risk to obtain a chromosomally abnormal child is higher than the risk of fetal loss due to the invasive technique.

Since the risk for chromosomal abnormalities increases with maternal age, prenatal diagnosis is recommended for women over 35 years of age. As a result, fewer children with Down syndrome are born from older women. However, 70% of the children with this chromosomal abnormality are born to women 35 years of age for whose prenatal diagnosis is not recommended. Non invasive prenatal diagnosis methods routinely used today for chromosomal abnormalities are recommended to low risk pregnancies. These methods include both biochemical and ultrasonographic markers which after algorithmic estimation are used only to evaluate the risk for chromosomal abnormalities. In cases with abnormal biochemical or ultrasonographic markers further investigation is recommended by invasive prenatal diagnosis. Additionally these markers are not specific for Trisomy 21 and are associated with a 5% false positive detection rate, leading to an increase in the number of women undergoing prenatal diagnosis.

New non-invasive techniques for prenatal diagnosis based on the isolation of fetal cells or cell free fetal DNA from maternal peripheral blood seem to be difficult to be applied into clinical practice due to lack of the specific characteristics to distinguish between fetal and maternal material. Nowadays, proteomic analysis is widely used for the detection of biological markers related to diseases. This technology can use all types of biological fluids and tissues as well as peripheral blood, leading to the development of fast, accurate and low cost clinical tests which can dramatically alter current diagnostic procedures and therapeutic approaches.

A limited number of published data on proteomic analysis of amniotic fluid and peripheral blood of pregnant women are related to the detection of proteins and peptides during gestation as a result of the molecular disturbances in DNA or RNA level [WO2005/0217 A (Pantarhei Bioscience BV [NL] et al)]. These studies show that proteomic analysis is a versatile approach for the detection of markers related to pathological conditions during pregnancy and these proteins/peptides can be used as markers of these conditions.

Studies of the brain from fetuses and adults with DOWN syndrome showed disturbances of enzymes participating in the metabolism of glucose, lipids, purins, folic acid and methionine, decreased N-methyltransferase of histamine, and disturbances in the expression of PRXIII of the brain, implying that more than one gene encodes for proteins related to the phenotype of the syndrome. In the present invention we detected and identified a protein, which as whole or fragments from their sequence, can be targets and be used originally as marker for the non-invasive prenatal diagnosis of Down syndrome, from all the women independently of risk for having a chromosomally abnormal fetus.

### D. DESCRIPTION OF THE INVENTION

Peripheral blood plasma samples obtained from pregnant women carrying fetuses with Trisomy 21 (Down syndrome) and from cases carrying chromosomally normal ones were analyzed using proteomic techniques as described in details below.

### Methods

Proteomic analysis includes proteins separation by two dimensional gel electrophoresis and protein identification by mass spectrometry. Protein identification procedure, apart from analytical techniques, includes specific software which is used to analyze two dimensional gels and to store obtained information into data bases.

### A. Protein separation by two dimensional ge/ electrophoresis.

The technique is based on the simultaneous separation of proteins by their charge and molecular weight resulting in the isolation within the gel of hundreds of proteins. Thus, such a study includes the protein separation by their charge (isoelectic focusing, IEF) followed by further separation based on their molecular weight.

The IEF is an equilibration procedure by which proteins move by the action of a high voltage electric field, across an acrylamide gel with differential pH, containing immobilines (IPG strips overlaid). During their movement across the strip proteins stabilize and focused on areas of the strip in which their charge is neutralized. The IPG stips are available in a great range of pH (such as 3-10) and size (7-24cm), as well as to more closed pH ranges (such as 4.5-5.5). IPG strips have the advantage to analyze a relatively high amount of sample. Electrophoresis conditions for IEF are related to the amount of the sample, the method used for the application of the sample on the strip, voltage and time of focusing.

At the second dimension, protein separation is based on the molecular weight. It takes place on constant or gradient polyacrylamide gel depending on the approximate size of proteins. Commonly constant 12% acrylamide and gradient 9-16% acrylamide gels are used for protein separation between 5-200kDa.

Finally gels are stained with dyes. Coumassie blue is usually selected when proteins have to be further identified by mass spectrometry.

Given the heterogeneity of proteins, each protein can be represented on the two dimensional gel by more than one spot, resulting in the identification of a higher number of gene products (proteins) than this of coded genes. In eukaryotics, it is known that 5-20 different proteins can be translated by the same gene.

### B. Mass spectrometry.

Following separation by two dimensional electrophoresis, proteins are identified either by 1) Mass Spectrometry (Matrix-assisted laser desorption-ionization, MALDI-TOF-MS) or 2) Ion spray techniques

Protein spots are excised from acrylamide gel, distained and treated with trypsin. All deriving peptides, from all spots are collected and analyzed by mass spectrometry and amino acid sequence determination of each peptide. Sophisticated software are used to compare the masses of detected peptides to the theoretical masses of all available protein data sets of all species. Protein identification is normally based on the detection of more than five peptides matching for each protein (up to twenty peptides are sometimes matched in cases of large protein molecules). Thus false detection rate is estimated to be less than 10⁻⁶.

Ion spray technique may provide safe results in amino acid sequencing of peptide with a probability of false rate less than 10⁻¹⁰, related to the number of peptides used for the analysis.

In conclusion, MALDI-TOF-MS technique allows rapid analysis of a large number of protein spots. However, ion spray technique is more reliable for protein identification. Those two techniques are usually used simultaneously and in tight correlation with each other in order to identify significant differences in protein expression between health and disease state and to determine their use as biological markers of therapeutic targets.

All technologies mentioned above, were used to analyze peripheral blood plasma samples obtained from pregnancies carrying fetuses with trisomy 21 (Down syndrome) and from cases carrying chromosomally normal ones **(IMAGE 1)**. The comparison of the two dimensional gels indicated a quantitative difference of the protein **Serum amyloid P-component (SAP, SAMP, SwissProt No: P02743) (IMAGE 2)**. Namely, in the plasma of women carrying fetuses with trisomy 21 (Down syndrome) the quantity of protein **Serum Amyloid P-component (SAP, SAMP, SwissProt No: P02743)** was found increased compared to the cases carrying chromosomally normal fetuses. The above result was further verified by Western Blot analysis using a special antibody **(IMAGE 3)**. These results suggested that the protein **Serum amyloid P-component (SAP, SAMP, SwissProt No: P02743)** could be a marker and could used for the prenatal check (diagnosis and/or evaluation) of the trisomy 21 (Down syndrome) with non-invasive methods. **Amyloid P component (AP)** is a naturally occurring glycoprotein that is found in serum and basement membranes. AP is also a component of all types of amyloid, including that found in individuals who suffer from Alzheimer's disease and Down's syndrome. The structure and function of active AP peptides will be useful for development of amyloid-targeted diagnostics and therapeutics (Heegaard N.H.H. et al: ligand-binding sites in human serum amyloid P component. Eur. J. Biochem. Vol. 239, p: 850-856, 1996).

That protein could detected in biological fluids by mass spectrometric immunoassay (MSIA) (US 2003/027216A1 (Kiernan Urban [US] et al) and recent studies suggested that the SAP protein could detected as diagnostic marker for cancer (US 2007/172900 A1, Cahill Michael [DE] et al). Recently our group has published the relevant paper about the detection of SAP in the maternal plasma of women carrying Down syndrome foetuses (Kolialexi A et al: Application of proteomics for the identification of differentially expressed protein markers for Down syndrome in maternal plasma. Prenatal Diagn. Vol 28, p: 691-698, 2008)

### E. ADVANTAGES OF THE CURRENT INVENTION

The methodology currently used for the prenatal diagnosis of fetal chromosomal abnormalities includes invasive and non-invasive techniques. Invasive procedures (amniocentesis, trophoblast biopsy and fetal blood) carry a significant risk for the mother and the fetus and for this reason they are recommended only in pregnant women in whom the risk to obtain a chromosomally abnormal child is higher than the risk of fetal loss due to the invasive technique.

Since the risk for chromosomal abnormalities increases with maternal age, prenatal diagnosis is recommended for women over 35 years of age. As a result, fewer children with Down syndrome are born from older women. However, 70% of the children with this chromosomal abnormality are born to women 35 years of age for whose prenatal diagnosis is not recommended. Non invasive prenatal diagnosis methods routinely used today for chromosomal abnormalities are recommended to low risk pregnancies. These methods include both biochemical and ultrasonographic markers which after algorithmic estimation are used only to evaluate the risk for chromosomal abnormalities. In cases with abnormal biochemical or ultrasonographic markers further investigation is recommended by invasive prenatal diagnosis. Additionally these markers are not specific for Trisomy 21 and are associated with a 5% false positive detection rate, leading to an increase in the number of women undergoing prenatal diagnosis.

New non-invasive techniques for prenatal diagnosis based on the isolation of fetal cells or cell free fetal DNA from maternal peripheral blood seem to be difficult to be applied into clinical practice due to lack of the specific characteristics to distinguish between fetal and maternal material.

In the current invention, the protein **Serum amyloid P-component (SAP, SAMP, SwissProt No: P02743)** was detected and identified in the peripheral blood plasma of women carrying fetuses with trisomy 21 (Down syndrome) compared to the maternal plasma of normal fetuses. That protein shown difference in the quantity in the maternal plasma of trisomy 21 fetuses compared to normal. That protein as whole molecule or the modified protein as a result of post-translation modifications, can be a target and be used originally as marker for non-invasive prenatal diagnosis of trisomy 21 (DOWN syndrome) from all women independently of the risk for having a chromosomally abnormal fetus in contrast to the available methods.

### F. IMAGE DESCRIPTION

**IMAGE 1.** Representative images of two dimensional polyacrylamide gels of peripheral blood plasma, produced by the application of the relevant methodologies. Each spot on the gel represents one protein. **(A)** Gel of maternal peripheral blood plasma from a women carrying normal fetus, **(B)** Gel of maternal peripheral blood plasma from a women carrying fetus with trisomy 21 (Down syndrome).

**IMAGE 2.** Comparison of two dimensional polyacrylamide gel of amniotic fluid obtained from gestation of a chromosomal normal embryo **(A)** and of two dimensional polyacrylamide gel obtained from a gestation of a Trisomy 21 pathological embryo **(B)**. 1A and 1 B indicate the regions of importance which further analysed in detail. Arrows indicated the spots showing differencies between the gels and in which **Serum amyloid P-component (SAP, SAMP, SwissProt No: P02743)** was identified by mass spectrometry.

**IMAGE 3**. Western blot analysis of the protein **Serum amyloid P-component (SAP, SAMP, SwissProt No: P02743)** in the maternal plasma of pregnant women. 10µg of total protein was electrophoresed and the detection of the protein **Serum amyloid P-component (SAP, SAMP, SwissProt No: P02743)** was effected by the SAP antibody clone E-14. Four maternal peripheral blood plasma from women carrying fetus with trisomy 21 (Down syndrome) (lanes 1-4) and four maternal plasma from women with normal embryo (lanes 5-8) were analyzed. **Serum amyloid P-component (SAP, SAMP, SwissProt No: P02743)** was found increased in the cases with trisomy 21 embryo compared to normal ones.

### G. DETAILED DESCRIPTION OF AN APPLICATION

Peripheral blood obtained from women from 15^{th} up to 18^{th} week of gestation is centrifuged in 3000 rpm for 15min in order to precipitate all cellular components and to obtain supernatant (plasma) for further analysis. The sample's supernatant can also be stored in -80°C until further process. Next, measurement of the protein concentration of the sample follows using Bradford assay and through nanocapillary electrophoresis in nano-cartridges using Bioanalyzer 2100 (Agilent Tech).

The protein **Serum amyloid P-component (SAP, SAMP, SwissProt No: P02743)** in the peripheral blood of pregnant women is possible to detected by Western blot. In that case, 10µg of total protein from maternal plasma electrophoresed in 12% acrylamide gel for 1h at 150V. The proteins transferred to a nitrocellulose membrane at 290mA for 1.5h at 4°C nad the membrane treated with the polyclonal antibody E-14 specific for the protein **Serum amyloid P-component (SAP, SAMP, SwissProt No: P02743),** diluted 1:200 in Tris Buffer Saline pH 7.4 with 5% fatty free milk for 18h at 4°C. Then the membrane treated with the a secondary antibody (dilution 1:70000) and the bound antibody was detected by chemiluminescence on an X-Ray film for different time of exposure.

In the case that a pregnant woman carries embryo with trisomy 21 the spot corelated to the protein **Serum amyloid P-component (SAP, SAMP, SwissProt No: P02743)** appeared to the X-Ray film more intense than in the cases with normal embryo (IMAGE 3).

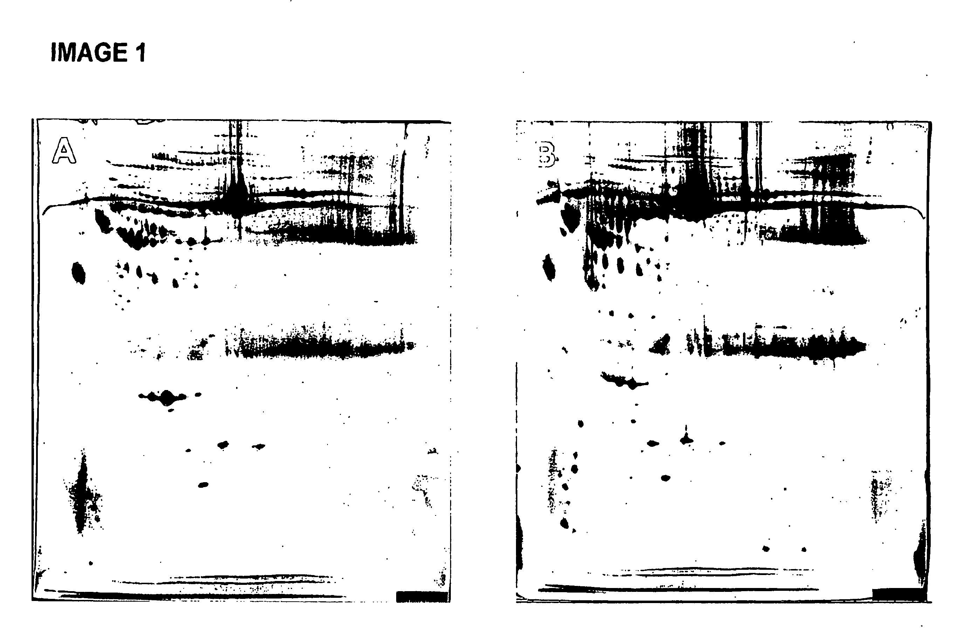

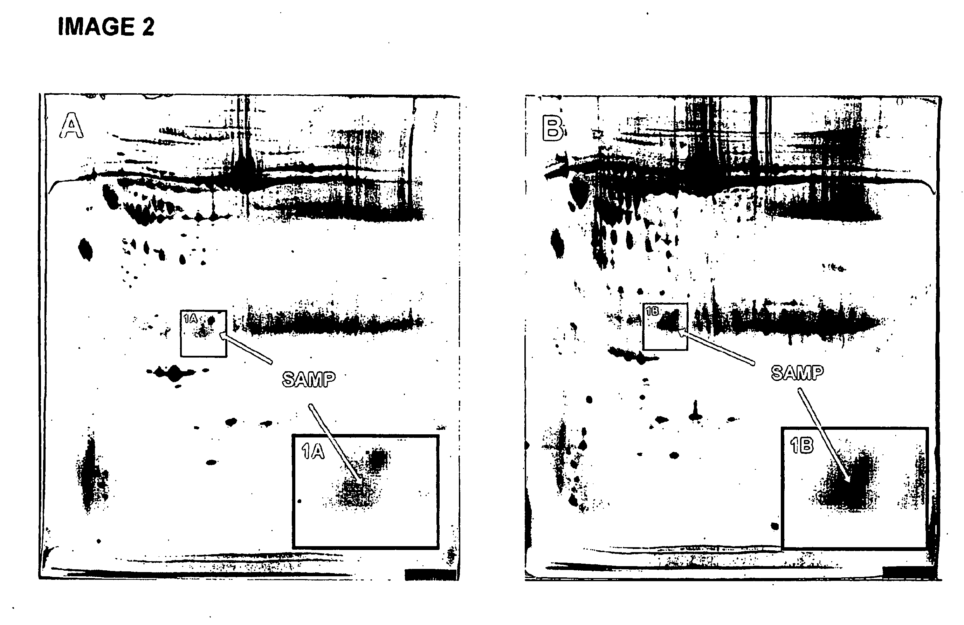

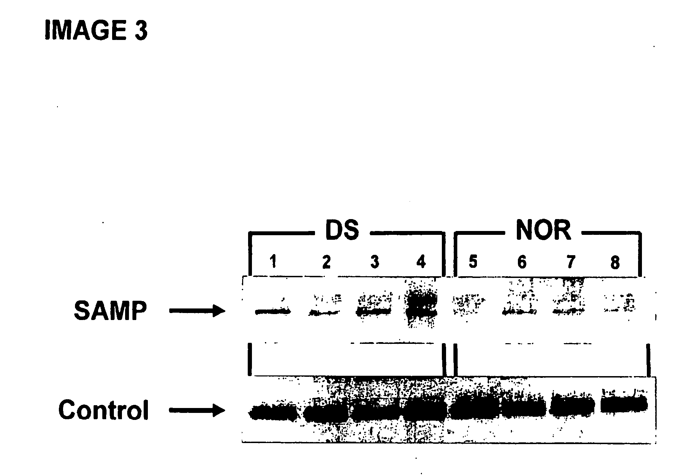

## Claims

1. A method for prognosis and/or diagnosis of Trisomy 21 (Down syndrome) in fetuses including:
**a.** The quantitative detection of the protein Serum amyloid P-component (SAP, SAMP, SwissProt No: P02743) in peripheral blood, serum and/or plasma from a pregnant woman and
**b.** The comparison of the quantitative value of that protein obtained in stage (a) with the quantitative value of that protein in the same biological fluid of women carrying chromosomically normal fetuses.
**c.** The prognosis and/or diagnosis is positive if the quantitative value of the protein Serum amyloid P-component (SAP, SAMP, SwissProt No: P02743), is higher than the quantitative value of that protein in the same biological fluid of women carrying chromosomically normal fetuses.

2. A method which, according to claim 1, is **characterized by** the detection of the whole Serum amyloid P-component (SAP, SAMP, SwissProt No: P02743) or of the modified protein as result of post-translation modifications.

3. A method which, according to claim 1 or 2, is **characterized by** the fact that the result for the prognosis and/or diagnosis is positive if the quantity of protein Serum amyloid P-component (SAP, SAMP, SwissProt No: P02743) or of the modified protein is higher than it's quantity mentioned as normal.

4. A method which, according to claims 1 to 3, is **characterized by** the fact that the result for the prognosis and/or diagnosis is positive if the quantity of protein Serum amyloid P-component (SAP, SAMP, SwissProt No: P02743) or of the modified protein is higher than it's quantity in the same biological fluid of women carrying normal embryo.

5. A method which, according to claims 1 to 4, is **characterized by** the fact that the result for the prognosis and/or diagnosis is positive if the quantity of protein Serum amyloid P-component (SAP, SAMP, SwissProt No: P02743) or of the modified protein is higher than the quantity of a reference protein.

6. A method which, according to each of the claims 1 to 5, is **characterized by** the fact that the quantitative analysis is performed using one of the following methods: nanotechnology, nanocapilary electrophoresis in a microchip, electrophoresis (of one or two dimensions), Western blot, microtitration (simple and/or multiple ELISA), chromatography (HPLC, LC, nanoLC, GC etc), mass spectrometry [peptide mass fingerprinting, post source decay, MALDI MS, MALDI MS-MS, LC-MS, LC-MS-MS, TANDEM-MS, TANDEM MS-MS, SELDI MS etc] or combination of these techniques.

7. A method which, according to each of the claims 1 to 6, is **characterized by** the fact that the quantitative analysis is performed using fluorescent substances or isotopes or antibodies or oligonucleotides or combination of these substances.

8. The use of the protein Serum amyloid P-component (SAP, SAMP, SwissProt No: P02743), for prognosis and/or diagnosis of Trisomy 21 (Down syndrome) during pregnancy.

9. The use, according to claim 8, of the protein Serum amyloid P-component (SAP, SAMP, SwissProt No: P02743) or of the modified protein, for prognosis and/or diagnosis of Trisomy 21 (Down syndrome) during pregnancy by non-invasive methods.

## Patentansprüche

1. Eine Methode zur Prognose und / oder Diagnose von Trisomie 21 (Down-Syndrom) bei Föten, welche:
**a.** Die quantitative Feststellung des Eiweißserums Amyloid-P-Komponente (SAP, SAMP, SwissProt Nr.: P02743) in peripherem Blut, Serum und / oder Plasma schwangerer Frauen und
**b.** Den Vergleich des quantitativen Werts dieses Eiweißes, das in diesem Stadium vorhanden ist, mit dem quantitativen Wert dieses Eiweißes in derselben biologischen Flüssigkeit von Frauen, welche im Hinblick auf die Chromosomen normale Föten austragen, beinhaltet.
**c.** Für den Fall, dass der quantitative Wert des Eiweißserums Amyloid-P-Komponente (SAP, SAMP, SwissProt Nr.: P02743) höher als der quantitative Wert dieses Eiweißes in derselben biologischen Flüssigkeit von Frauen, welche im Hinblick auf die Chromosomen normale Föten austragen, ist, ist die Prognose und / oder die Diagnose positiv.

2. Eine Methode gemäß Anspruch 1, die durch die Feststellung des gesamten Serums Amyloid-P-Komponente (SAP, SAMP, SwissProt Nr. P02743) oder durch das veränderte Eiweiß als Ergebnis einer posttranslationalen Modifikation **gekennzeichnet** ist.

3. Eine Methode gemäß den Ansprüchen 1 oder 2, die durch die Tatsache, dass das Ergebnis der Prognose und / oder die Diagnose für den Fall, dass die Menge des Eiweißserums Amyloid-P-Komponente (SAP, SAMP, SwissProt Nr.: P02743) oder des veränderten Eiweißes höher als die Menge, die als normal betrachtet wird, ist, positiv ist, **gekennzeichnet** ist.

4. Eine Methode gemäß den Ansprüchen 1 bis 3, die durch die Tatsache, dass das Ergebnis der Prognose und / oder der Diagnose für den Fall, dass die Menge des Eiweißserums Amyloid-P-Komponente (SAP, SAMP, SwissProt Nr.: P02743) oder des veränderten Eiweißes höher als seine Menge in derselben biologischen Flüssigkeit von Frauen, die einen normalen Embryo austragen, ist, positiv ist, **gekennzeichnet** ist.

5. Eine Methode gemäß den Ansprüchen 1 bis 4, die durch die Tatsache, dass das Ergebnis für die Prognose und / oder die Diagnose für den Fall, dass die Menge des Eiweißserums Amyloid-P-Komponente (SAP, SAMP, SwissProt Nr.: P02743) oder des veränderten Eiweißes höher als die Menge des Referenzeiweißes ist, positiv ist, **gekennzeichnet** ist.

6. Eine Methode gemäß jeden Anspruchs von 1 bis 5, die durch die Tatsache, dass die quantitative Analyse unter Verwendung einer der folgenden Methoden durchgeführt wurde, **gekennzeichnet** ist:
Nanotechnologie, Nanokapillar-Elektrophorese in einem Mikrochip, Elektrophorese (von einer oder zwei Dimensionen), Western Blot, Mikrotiter (einfaches und / oder mehrfaches ELISA-Verfahren), Chromatografie (HPLC, LC, Nano-LC, GC etc.), Massenspektrometrie [Peptide-Mass-Fingerprinting, Post-Source-Decay, MALDI MS, MALDI MS-MS, LC-MS, LC-MS-MS, TANDEM-MS, TANDEM MS-VS, SELDI MS etc.) oder eine Kombination dieser Techniken.

7. Eine Methode gemäß eines jeden Anspruchs von 1 bis 6, die durch die Tatsache, dass eine quantitative Analyse unter Verwendung fluoreszierender Substanzen oder Isotopen oder Antikörpern oder Oligonukleotiden oder einer Kombination dieser Substanzen durchgeführt wird, **gekennzeichnet** ist.

8. Die Verwendung des Eiweißserums Amyloid-P-Komponente (SAP, SAMP, SwissProt Nr. P02743) für die Prognose und / oder Diagnose von Trisomie 21 (Down Syndrom) während der Schwangerschaft.

9. Die Verwendung gemäß Anspruch 8 des Eiweißserums Amyloid-P-Komponente (SAP, SAMP, SwissProt Nr. P02743) oder des veränderten Eiweißes für die Prognose und / oder Diagnose von Trisomie 21 (Down Syndrom) durch nicht invasive Methoden während der Schwangerschaft.

## Revendications

1. Méthode pour le pronostic et / ou le diagnostic de la trisomie 21 (syndrome de Down) chez le foetus :
**a.** Détection quantitative de la protéine amyloïde P sérique (SAP SAMP SwissProt No P02743) dans le sang périphérique, sérum et / ou plasma pour femme enceinte et
**b.** La comparaison de la valeur quantitative de protéine obtenue à la phase (a) et de la valeur quantitative de protéine dans le même fluide biologique pour une femme portant des foetus aux chromosomes normaux.
**c.** Le pronostic et / ou le diagnostic est positif lorsque la valeur quantitative de protéine amyloïde P sérique (SAP, SAMP, SwissProt No P02743) est supérieure à la valeur quantitative de cette protéine dans le même fluide biologique d'une femme portant des foetus aux chromosomes normaux.

2. Une méthode qui, selon la revendication 1, est **caractérisée par** la détection du sérum amyloïde P (SAP, SAMP, SwissProt No P02743) ou de la protéine modifiée comme résultat de la modification post-traductionnelle.

3. Méthode qui, selon les revendications 1 ou 2, est **caractérisée par le fait que** le résultat du pronostic et / ou du diagnostic est positif lorsque la quantité de protéine amyloïde P sérique (SAP, SAMP, SwissProt No P02743) ou de la protéine modifiée est supérieure à la quantité dite normale.

4. Méthode qui, selon les revendications 1 à 3, est **caractérisée par le fait que** le résultat du pronostic et / ou du diagnostic est positif lorsque la quantité de protéine amyloïde P sérique (SAP, SAMP, SwissProt No P02743) ou de la protéine modifiée est supérieure à la quantité de cette protéine dans le même fluide biologique d'une femme portant un embryon normal.

5. Méthode qui, selon les revendications 1 à 4, est **caractérisée par le fait que** le résultat du pronostic et / ou du diagnostic est positif lorsque la quantité de protéine amyloïde P sérique (SAP, SAMP, SwissProt No P02743) ou de la protéine modifiée est supérieure à la quantité d'une protéine de référence.

6. Méthode qui, selon les revendications 1 à 5, est **caractérisée par le fait que** l'analyse quantitative est effectuée par l'une des méthodes suivantes : nanotechnologie, électrophorèse nanocapillaire dans une micropuce, électrophorèse (d'une ou deux dimensions), immuno-empreinte, microtitration (simple et / ou multiple, méthode ELISA), chromatographie (chromatographie liquide à haute performance, chromatographie en phase liquide (CL), nano-chromatographie, chromatographie gazeuse, etc...), MS - spectrométrie de masse (empreinte digitale de masse de peptide, Post Source Decay, méthode MALDI-MS, CL-MS, CL-MS-MS, MS en tandem, MS-MS en tandem, MS SELDI, etc...) ou la combinaison de ces techniques.

7. Méthode qui, selon les revendications 1 à 6, est **caractérisée par le fait que** l'analyse quantitative est effectuée avec utilisation soit de substance fluorescente, d'isotopes, d'anticorps, d'oligonucléotides ou doit de la combinaison de ces substances.

8. L'utilisation de la protéine amyloïde P sérique (SAP, SAMP, SwissProt No P02743) pour le pronostic et / ou le diagnostic de la trisomie 21 (syndrome de Down) pendant la grossesse.

9. L'utilisation, selon la revendication 8, de la protéine amyloïde P sérique (SAP, SAMP, SwissProt No P02743) ou de la protéine modifiée pour le pronostic et / ou le diagnostic de la trisomie 21 (syndrome de Down) pendant la grossesse par des examens non-invasifs.
